# EUROPEAN PATENT APPLICATION

(11) **EP 1 958 594 A1**
(43) Date of publication of application: **20.08.2008**
(21) Application number: 08101441.7
(22) Date of filing: 08.02.2008
(51) Int. Cl.: A61F 2/16

(54) **Lens delivery system**

(30) Priority: 15.02.2007 US 675402
(71) Applicant: ALCON RESEARCH, LTD., 76134-2099 TX, Texas Fort Worth (US)
(72) Inventor: Downer, David Anthony, Fort Worth, TX 76137 (US)
(74) Representative: Hanna, Peter William Derek

(57) **Abstract**

A lens delivery system is described having a plunger rod (26) with a plurality of elastomeric wipers (30) that frictionally and slidably engage the interior bore (32) of the delivery system handpiece (14), or the elastomeric wipers (30') can be located on the bore (32) and frictionally and slidably engage the plunger rod (26). The amount of frictional engagement by the wipers can be varied and controlled by adjusting the geometry, size or material properties (modulus of elasticity) of the wipers. The wipers help to provide feedback to the user, and also help provide more even, consistent force throughout the full range of travel of the plunger rod.

## Description

This invention relates to intraocular lenses (IOLs) and more particularly to devices used to inject IOLs into an eye.

### Background of the Invention

The human eye in its simplest terms functions to provide vision by transmitting and refracting light through a clear outer portion called the cornea, and further focusing the image by way of the lens onto the retina at the back of the eye. The quality of the focused image depends on many factors including the size, shape and length of the eye, and the shape and transparency of the cornea and lens.

When trauma, age or disease cause the lens to become less transparent, vision deteriorates because of the diminished light which can be transmitted to the retina. This deficiency in the lens of the eye is medically known as a cataract. The treatment for this condition is surgical removal of the lens and implantation of an artificial lens or IOL.

While early IOLs were made from hard plastic, such as polymethylmethacrylate (PMMA), soft, foldable IOLs made from silicone, soft acrylics and hydrogels have become increasingly popular because of the ability to fold or roll these soft lenses and insert them through a smaller incision. Several methods of rolling or folding the lenses are used. One popular method is an injector cartridge that folds the lenses and provides a relatively small diameter lumen through which the lens may be pushed into the eye, usually by a soft tip plunger. One of the most commonly used injector cartridge design is illustrated in U.S. Patent No. 4,681,102 (Bartell), and includes a split, longitudinally hinged cartridge. Another recently introduced disposable lens delivery system is disclosed in USPN 7,156,854 B2 (Brown, et al.). Other cartridge designs are illustrated in U.S. Patent Nos. 5,494,484 and 5,499,987 (Feingold) and 5,616,148 and 5,620,450 (Eagles, et al.). In an attempt to avoid the claims of U.S. Patent No. 4,681,102, several solid cartridges have been investigated, see for example U.S. Patent No. 5,275,604 (Rheinish, et al.) and 5,653,715 (Reich, et al.).

The ability to express a lens out of a cartridge without damage is dependent on lens design and material. Silicone lenses, being made from a relatively rugged and durable material, can be compressed more aggressively. High water content hydrogel material, being more fragile, must be folded more gently. Soft acrylics, being viscoelastic in nature, are highly sensitive to temperature, and can be brittle if too cold, and can be unworkable if too warm. Soft acrylics, when compressed at an appropriate temperature, can be described as "flowing" rather than folding. For this reason, soft acrylics are best compressed slowly and in a very controlled manner.

Prior art lens delivery handpieces suitable for use with soft acrylic IOLs generally have plungers that are either advanced by use of a screw or by pushing on the proximal end of the plunger, similar to the motion used with a syringe. Handpieces having plunger advanced by a screw generally advance the lens in an even, controlled manner. Syringe type plunger advancement can be problematic because the tactic feedback from the plunger varies. Initial pressure must be relatively high to overcome the initial resistance of the lens. Once the lens is moving, the required pressure on the Plunger rod to continue to advance the lens will lessen, but then increases gradually as the lens is folded more tightly and enters the narrow, restricted nozzle bore. As a result, it can be difficult to apply slow, even and steady pressure to the plunger rod in syringe-type lens delivery systems.

Accordingly, a need continues to exist for a device to help provide even, consistent tactile feedback to the user of a syringe-type lens injection system.

### Brief Summary of the Invention

The present invention improves upon prior art by providing a lens delivery system in accordance with claims which follow, having a plunger rod with a plurality of elastomeric wipers that frictionally and slidably engage the interior bore of the delivery system handpiece, or the elastomeric wipers can be located on the bore and frictionally and slidably engage the plunger rod. The amount of frictional engagement by the wipers can be varied and controlled by adjusting the geometry, size or material properties (modulus of elasticity) of the wipers. The wipers help to provide feedback to the user, and also help provide more even, consistent force throughout the full range of travel of the plunger rod.

It is accordingly an objective of the present invention to provide a lens delivery system that is suitable for folding lenses made from a soft acrylic material. It is a further objective of the present invention to provide a lens delivery system having a plurality of internal elastomeric wipers.

It is a further objective of the present invention to provide a lens delivery system that provides a more even, consistent force throughout the full range of travel of the plunger rod.

Other objectives, features and advantages of the present invention will become apparent with reference to the drawings, and the following description of the drawings and claims.

### Brief Description of the Drawings

FIG. 1 is a top plan view of the lens delivery system of the present invention.
FIG. 2 is an enlarged partial top plan view of one embodiment of the lens delivery system of the present invention taken at circle 2 in FIG. 1.
FIG. 3 is an enlarged partial perspective view of one embodiment of a plunger that may be used with the lens delivery system of the present invention.

### Detailed Description of the Preferred Embodiments

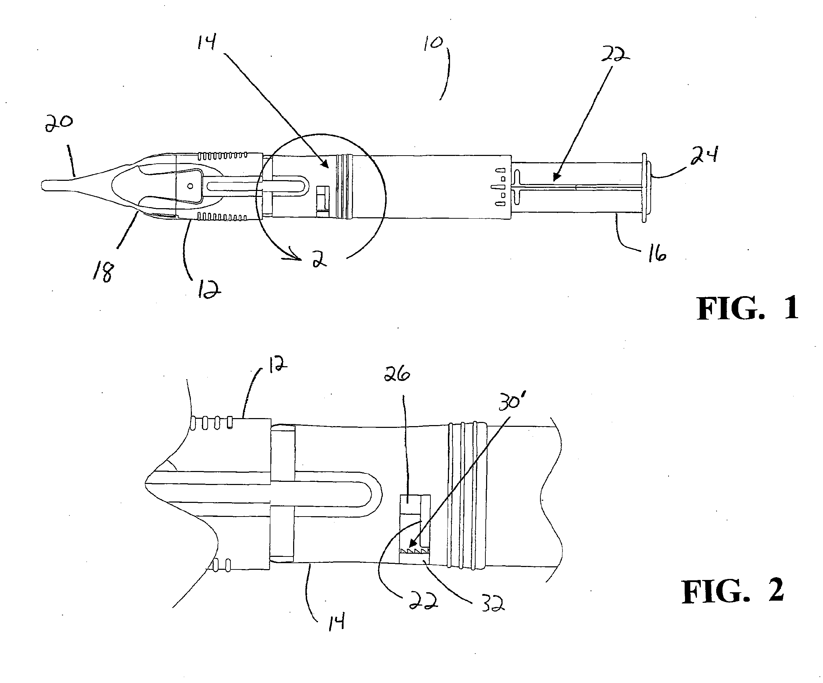

As best seen in FIG. 1, lens delivery system 10 of the present invention generally includes lens delivery cartridge 12, tubular handpiece 14 and plunger 16. Handpiece 14 may be of any suitable construction, such as molded thermoplastic or machined aluminum, stainless steel or titanium. Cartridge 12 generally has tubular body 18 and injection nozzle 20. Cartridge 12 is molded as a single piece from any suitable thermoplastic, such as polypropylene, and the thermoplastic may contain a lubricity enhancing agent such as those disclosed in U.S. Pat. No. 5,716,364. Alternatively, cartridge 12 may be integrally formed with handpiece 14. Nozzle 20 preferably is round, oval or elliptical in cross-section and has a cross-sectional area of between around 1.0 mm² to around 2.6 mm². An intraocular lens (not shown) is pushed out of cartridge 12 and down nozzle 18 by plunger 16 in a manner well-known in the art.

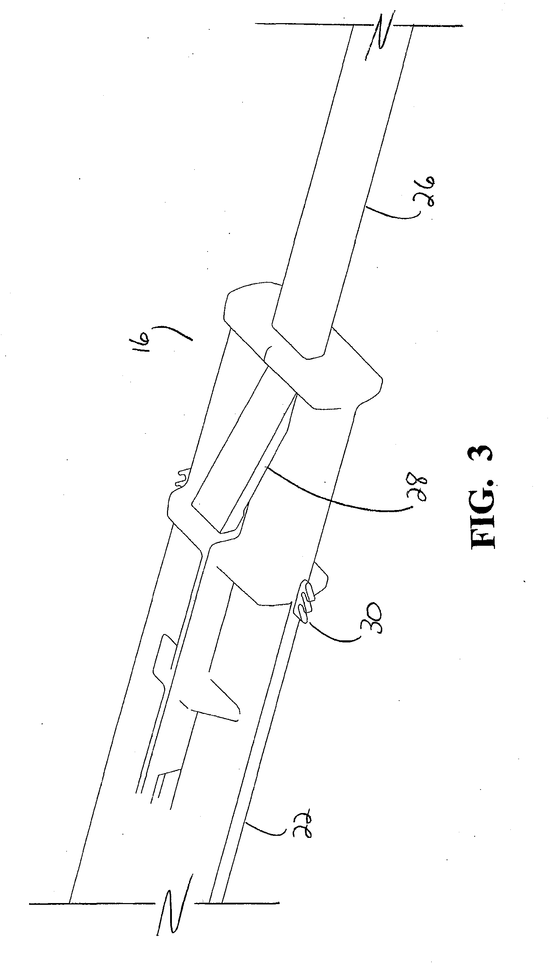

As best seen in FIG. 3, plunger 16 generally contains a proximal thickened portion 22 having proximal cap 24 and distally extending plunger rod 26. Plunger rod 26 is thinner than thickened portion 22 and sized and shaped to reciprocate within nozzle 20. Plunger 16 is sized and shaped to reciprocate within handpiece 14 and may contain additional features, such as stop 28 to help prevent movement of plunger 16 within handpiece 14 so that, for example, pushing on cap 24 propels plunger rod into and down nozzle 20 while stop 28 prevents plunger rod 26 from being drawn back out of nozzle 20.

Thickened portion 22 of plunger 16 may contain a plurality of elastomeric wipers 30 that frictionally engage the interior wall of tubular body 14. Such frictional engagement increases the force required to push plunger 16 within tubular body 14. One skilled in the art will recognize that the amount of force can be controlled by increasing the number and/or size of wipers 30 or by varying the material properties, such as modulus of elasticity, of wipers 30. Although wipers 30 increase the force required to push plunger 16 within tubular body 14, wipers 30 also help to provide feedback to the user, and help provide more even, consistent force throughout the full range of travel of plunger 16. Wipers 30 can be attached to plunger 16 in any suitable manner, such as over-molding. Preferably, wipers 30 extend only partially along or around thickened portion 22 of plunger 16.

Alternatively, as seen in FIGS. 1 and 2, wipers 30' can be attached to interior wall 32 of tubular handpiece 14. Wipers 30' are of similar construction as wipers 30 and attached to interior wall 32 of tubular handpiece 14 in a manner similar to that used to attach wipers 30 to plunger 16.

While certain embodiments of the present invention have been described above, these descriptions are given for purposes of illustration and explanation. Variations, changes, modifications and departures from the systems and methods disclosed above may be adopted without departure from the scope of the present invention.

## Claims

1. An intraocular lens delivery system (10), comprising:
a) a cartridge (12);
b) a tubular handpiece (14) having an interior wall (32);
c) a plunger (16) having a proximal thickened portion (22), to which is attached a distally extending plunger rod (26), the plunger being sized and shaped to reciprocate within the tubular handpiece;
**characterized by**
d) at least one wiper (30) attached to the thickened portion (22) of the plunger (16) so as to frictionally engage the interior wall (32) of the tubular handpiece, or
d') at least one wiper (30') attached to the interior wall (32) of the tubular handpiece so as to frictionally engage the thickened portion (22) of the plunger (16).

2. The lens delivery system of claim 1, wherein the wiper (30,30') is made from an elastomeric material.
